# EUROPEAN PATENT APPLICATION

(11) **EP 3 875 039 A1**
(43) Date of publication of application: **08.09.2021**
(21) Application number: 20305221.2
(22) Date of filing: 02.03.2020
(51) Int. Cl.: A61B 17/04

(54) **NEEDLE, DEVICE AND SYSTEM FOR SUTURING AN INCISION IN TISSUES OF A PATIENT**

(71) Applicant: CHU de Nice, 06000 Nice (FR)
(72) Inventor: Sejor, Eric, 06000 Nice (FR)
(74) Representative: Osha Liang

(57) **Abstract**

The disclosure relates to a needle (11) and a device (100) for suturing an incision in tissues of a patient. The present disclosure also relates to a system (200) and a method for suturing an incision in tissues (60) of a patient. The distal end portion comprises a beveled tip (13) for cutting through tissues (60) of a patient and a longitudinal slit (14) extending from the beveled tip (13) for a predetermined length.

## Description

### Field of the disclosure

The present disclosure relates to a needle, a device and a system for suturing an incision in tissues of a patient. The present disclosure also relates to a method for suturing an incision in tissues of a patient.

### Technical background

Minimally invasive surgery is a surgery technique which has replaced many standard invasive surgical operations requiring large incisions in tissues. In this surgery technique, access to the surgical field is made through small incisions (generally 5-18 mm in diameter). Trocars are then inserted through the incision to permit the introduction of a laparoscope and surgical instruments which can be manipulated by a surgeon while viewing the surgical field on a monitor. Minimally invasive surgery affords the patient considerably less pain and disfigurement, and, as a consequence, a much faster recovery. The rapid return of the patient to productive activity further reduces the ultimate cost of the surgery.

While trocars are widely used in minimally invasive surgery, such use creates several clinical problems, such as incisional hernia. The small size of the incision makes the suture of the deep part of the incision problematic and time consuming. As a consequence, most of surgeons do not suture the deep part of the incision. On the other hand, when the suture of the deep part of the incision is performed, this requires either direct visualization, whenever a direct visualization is possible, or internal visual assistance (laparoscopy). For example, one known method requires the introduction of a suture needle in the abdominal cavity. The surgeon sees the suture needle via a laparoscope as the suture needle pierces the abdominal wall. The surgeon then grasps several suture threads around the surgical incision, and then ties off the suture threads.

Since the surgeon cannot directly visualize the exact position of the suture needle until the suture needle has passed completely through the abdominal wall, several insertions may be required in order to place the suture needle at an ideal and proper distance from the trocar incision.

A predetermined distance from the suture needle location to the original incision should be respected because the suture needle must be far enough from the trocar incision to secure an optimal amount of abdominal wall tissue. If the suture needle distance from the incision is too small, an insufficient amount of tissue will be secured with a consequent risk of inadequate suture of the surgical defect. However, if the suture needle distance from the point of the original trocar incision is too great, incision suture will result in excessive tissue being grasped, and the patient will be left with an unsightly "knot" of tissue.

From prior art document US 2015201920 A1 a system is known comprising a tubular body configured to be introduced through an incision and having a distal end and a proximal end and a length between the distal and proximal ends sized so that the distal end may be positioned adjacent the innermost tissue layer while the proximal end is accessible outside the patient. The tubular body further comprises at least one stabilizer element movably supported at the distal end of the tubular body suitable for contacting an interior surface of an innermost tissue layer.

From prior art document US 2015157316 A1 a laparoscopic fascial closure system is also known which may be used with a suture grasper retriever to capture a portion of a suture with a template disposed within a body cavity and then facilitate free movement or sliding of the suture at or within a device tip during retraction of the system from the body cavity.

However, such prior art systems still present a risk of pinching a patient's organs, especially when a tip, a stabilizer or an element equivalent thereto is deployed inside a body cavity of a patient and pull back to contact an interior surface of an innermost tissue layer. In such a situation, organs could be damaged.

### Summary of the disclosure

In what follows, the term "comprise" is synonym of (means the same as) "include" and "contains", is inclusive and open and does not exclude other non-recited elements.

According to a first aspect, the present disclosure relates to a hollow needle comprising a proximal end portion and a distal end portion, wherein the distal end portion comprises a beveled tip for cutting through tissues of a patient and a longitudinal slit extending from the beveled tip for a predetermined length.

In the present description and the following claims, the terms "distal end" are used to indicate a component which is the end closest to the patient, in use, e.g., the end of the component intended to touch or enter a tissue first. For example, the distal end of the needle is the piercing end thereof. In the present description and the following claims, the terms "proximal end" are used to indicate the end of a component oriented opposite the distal end.

As described in more details in embodiments of a device and a system for suturing an incision in tissues of a patient, the above-mentioned needle is suitable to be used in such a device and system without inducing wounds in organs or tissues situated below the tissues to be sutured, such as, for example, lungs, heart, stomach, gastrointestinal tract and vessels.

According to one or more embodiments, the beveled tip of the needle is configured to cut through body tissues, such as for example abdominal wall tissues. However, other types of tissues may also be cut by such a needle, such as, for example, fat tissues, connective tissues, and muscular tissues.

According to one or more embodiments, the beveled tip is formed by a single planar cut through the needle at a predetermined angle to the longitudinal axis of the needle. This angle may be comprised from 10° to 45°, for example from 15° to 40°, for example from 25° to 35°.

According to one or more embodiments, the longitudinal slit extends from a distal end of the beveled tip over a predetermined length of the needle, for example for at least 10%, for example from 10% to 50%, 15% to 45%, 20% to 40%, of the length thereof.

According to one or more embodiments, the needle comprises a ring located at a predetermined length of the needle in a region other than the region including the longitudinal slit, for example located at half length of the needle. The ring is intended to cooperate with an element of a device for suturing an incision in tissues of a patient, which will be described in more detail in the following, for setting the needle at a predetermined tissue penetration depth. In this manner, the preservation of the tissues and organs located below this predetermined tissue penetration depth is ensured. Also, the safety during use of the needle by the surgeon is improved.

According to one or more embodiments, the ring comprises a hollow cylinder having a truncated cone shape. In these embodiments, the ring is intended to cooperate with a device for suturing an incision in tissues of a patient comprising a hollow cylinder internally configured as a truncated cone shape complementary to the truncated cone shape of the ring.

According to a second aspect, the present disclosure relates to a device for suturing an incision in tissues of a patient, the device comprising the hollow needle according to any of the embodiments herein described, a first cylindrical element configured to be received within the hollow needle in a slidable manner, and a guiding means for sliding the first cylindrical element within the hollow needle. The first cylindrical element comprises a smooth, for example rounded, distal end. The smooth distal end may be suitable for protecting tissue from injury by the beveled tip. Thanks to this configuration, the device for suturing an incision in tissues of a patient may be easily operated by a surgeon without causing any injury to the tissues and organs underlying the tissues to be sutured.

According to one or more embodiments, the guiding means comprises an elastic means such as a spring, for example a flat wound spring or a coil spring.

According to one or more embodiments, the guiding means comprises a elastic-means loaded piston or plunger, for example a spring loaded piston or plunger.

According to one or more embodiments, the first cylindrical element is hollow and the device further comprises a second cylindrical element configured to slide within the first cylindrical element and optionally to be automatically operated.

In the present description and the following claims, the automatic operation of the second cylindrical element may refer to a repetitive movement of the second cylindrical element activated by a device, such as, for example, a tag gun.

In this manner, the second cylindrical element can expel at least one suture thread carried by the first cylindrical element, for example by manually pushing the second cylindrical element. However, it is also possible to automatically push the second cylindrical element by means of automatic means. In the case of an automatic suturing operation, the device can anchor a plurality of suture threads in a precise manner and in a short time.

According to one or more embodiments, the device further comprises a suture thread. According to one or more embodiments, the suture thread comprises one end having a T-shape end. Using a suture thread comprising one end having a T-shape permits to anchor the T-shape end in tissues of a patient, thus avoiding the need to exit from the tissue through other parts of the tissues.

According to one or more embodiments, the first cylindrical element comprises a longitudinal slit extending from the rounded distal end for a predetermined length and the suture thread is inserted through this longitudinal slit.

According to one or more embodiments, the device further comprises a tubular hollow body comprising at least one guide channel for guiding the hollow needle therethrough at a predetermined angle with respect to a longitudinal axis of the tubular hollow body, the at least one guide channel extending from a first opening to a second opening of the tubular hollow body in a portion proximal to a distal end of the tubular hollow body. Accordingly, the hollow needle may be inserted in at least one guide channel and guided to a desired cutting position in a simple and precise manner.

According to one or more embodiments, the predetermined angle at the hollow needle is guided is comprised from 10° to 45°, for example from 15° to 40°, for example from 25° to 35°.

According to one or more embodiments, the device comprises at least two guide channels, for example opposite to one another with respect to the longitudinal axis of the tubular hollow body.

According to one or more embodiments, the guide channels may be straight channels and, for example, may have distinct diameters to guide needles of distinct size.

According to one or more embodiments, the two guide channels cross each other, thus sharing a common section.

According to one or more embodiments, the two guide channels are separate from each other, for example are parallel to one another or arranged at respective predetermined angles with respect to the longitudinal axis of the tubular hollow body. According to one or more embodiments, these predetermined angles may differ of at least 5°, for example from 5° to 15°, for example from 7° to 10°.

According to one or more embodiments, the tubular hollow body is configured to receive a surgical instrument, such as, for example, a laparoscope or any miniaturized surgical instrument suitable for minimally invasive surgery, such as for example a grasper.

According to one or more embodiments, for example when the device is not intended to receive a surgical instrument, the tubular hollow body comprises a smooth, for example rounded, distal end.

According to one or more embodiments the tubular hollow body comprises a means for uplifting one or more tissue layers.

According to one or more embodiments, the means for uplifting one or more tissue layers comprises an inflatable means, which may be for example located at the distal end of the tubular hollow body.

According to one or more embodiments, the inflatable means comprises two balloons, which may be for example located on opposite sides of the distal end of the tubular hollow body. The two balloons may be arranged on a plane perpendicular to the longitudinal axis of the tubular hollow body. Accordingly, it is possible to preserve tissues and organs from injury in a simple and reliable manner and to prevent damages to the balloons.

According to one or more embodiments, when the device comprises inflatable means, the device may further comprise pressurizing means for inflating the inflatable means. According to one or more embodiments, the pressurizing means comprises a piston manually activated to compress the air or gas trapped between the piston and the proximal end of the tubular hollow body. The pressuring means may be, for example, located at a proximal end of the tubular hollow body.

According to one or more embodiments, the pressurizing means comprises means for maintaining the inflatable means inflated, such as, for example, a locking system.

According to one or more embodiments, the device further comprises a trocar receiving the device. For example, the trocar may be inserted in the incision to be sutured.

According to one or more embodiments, the device further comprises a latch arranged on an external wall of the tubular hollow body, for example in a retractable manner. In this manner, the device may be blocked in the trocar. According to one or more embodiments, the trocar may be also provided with a corresponding supporting latch.

According to a third aspect, the present disclosure relates to a system for suturing an incision in tissues of a patient, comprising the device according to any embodiment of the present disclosure, and a means for automatically sliding the second cylindrical element within the first cylindrical element.

Accordingly, a suture thread can be automatically expelled from the system for suturing an incision in tissues of a patient in order to be anchored in tissue layers. As a consequence, the required time to anchor a suture thread is reduced and the gesture of the surgeon is reproducible and therefore safer.

According to one or more embodiments, the means for automatically sliding the second cylindrical element within the first cylindrical element comprises a projection mechanism for projecting the second cylindrical element, which is housed in a slidable manner within the first cylindrical element for surgical suture gripping. For example, the projection mechanism may comprise a tag gun similar to a tag gun for using and reloading with tags in the clothes industry. For example, the tag gun may comprise a gear for converting an initial movement of a gun trigger into a sliding movement of the second cylindrical element within the first cylindrical element, as well as a spring for returning the second cylindrical element to a resting position.

According to a fourth aspect, the present disclosure relates to a tubular hollow body comprising at least one guide channel for guiding a needle therethrough, such as the hollow needle described above, at a predetermined angle with respect to a longitudinal axis of the tubular hollow body, the at least one guide channel extending from a first opening to a second opening of the tubular hollow body in a portion proximal to a distal end of the tubular hollow body. According to one or more embodiments of the fourth aspect, the tubular hollow body may comprise any of the features defined above with reference to the tubular hollow body making part of the device for suturing an incision in tissues of a patient.

### Brief description of the drawings

The present disclosure will be better understood and other advantages and embodiments will become clear on reading the description that follows, given purely by way of indication and in no way limiting, and by referring to the appended figures in which:
- FIGS. 1A and 1B are front views of a needle according to respective embodiments of the present disclosure.
- FIG. 1C is a side view of the needle of FIG. 1A comprising an exemplary ring cooperating therewith.
- FIG. ID shows a side view, a perspective view and a cross-sectional view of the ring shown in FIG. 1C.
- FIG. 2A is a side view of a device for suturing an incision in tissues of a patient according to a first embodiment of the present disclosure.
- FIG. 2B is a side view of a device for suturing an incision in tissues of a patient according to a second embodiment of the present disclosure in a resting position.
- FIG. 2C is a side view of the device for suturing an incision in tissues of a patient according to the second embodiment of the present disclosure in a cutting position.
- FIGs. 3A and 3B are a front and a back view, respectively, of a tubular hollow body of a device for suturing an incision in tissues of a patient according to one or more embodiments of the present disclosure.
- FIG. 4 is a side view of a portion of a means for automatically operating a device for suturing an incision in tissues of a patient according to one or more embodiments of the present disclosure.
- FIGs. 5 and 5bis show successive perspective views illustrating steps of a method for suturing an incision in tissues of a patient according to one or more embodiments of the present disclosure.
- FIG. 6 shows a perspective view of a step of the method for suturing an incision in tissues of a patient according to one or more embodiments of the present disclosure, at which tissues are uplifted.

### Detailed description of embodiments

Embodiments of the present disclosure will now be described in detail with reference to the accompanying figures. In the following detailed description of embodiments of the present disclosure, numerous specific details are set forth in order to provide a more thorough understanding of the present disclosure. However, it will be apparent to one of ordinary skill in the art that the present disclosure may be practiced without these specific details. In other instances, well-known features have not been described in detail to avoid unnecessarily complicating the description.

The following description provides a particular, non-limiting example of a needle, a device, and a system for suturing an incision in tissues of a patient as well as of a method for suturing an incision in tissues of a patient.

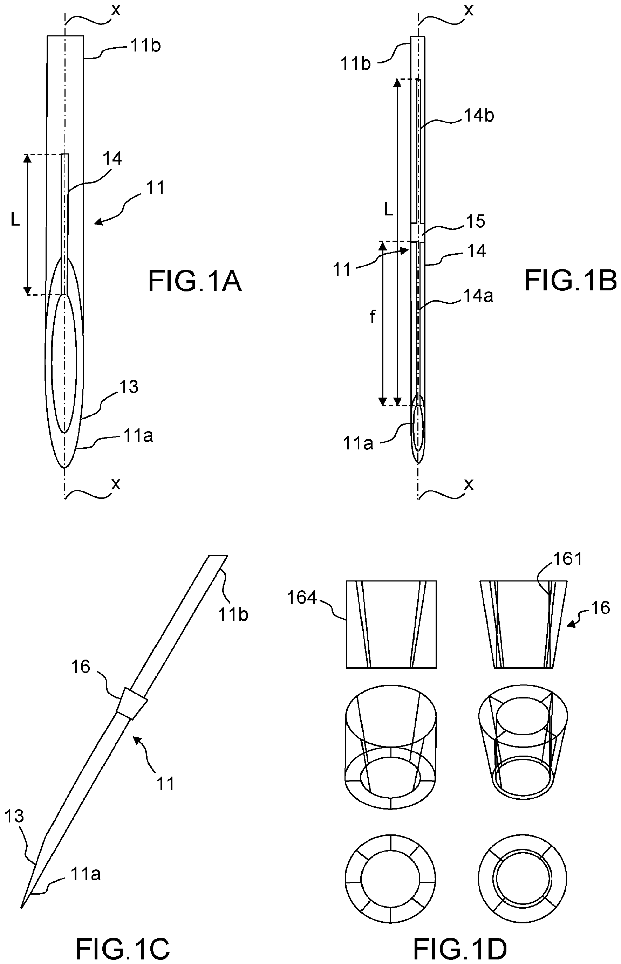

Embodiments of a needle, designated by reference number 11, are shown in Figs. 1A and 1B. In each embodiment, the needle 11 may be used for cutting through tissues, such as for example abdominal wall tissues. The needle 11 is hollow and comprises a proximal end portion 11b and a distal end portion 11a. The distal end portion 11a comprises a beveled tip 13 for cutting through tissues of a patient and a longitudinal slit 14 extending from the beveled tip 13 for a predetermined length L. According to one or more embodiments, the predetermined length L may be of at least 10% of the length of the needle 11. For example, the predetermined length L may correspond to 10% to 50% of the length of the needle 11. In Fig. 1A, for example, the longitudinal slit 14 extends from the beveled tip 13 for 10% of the length of the needle 11. In Fig. 1B, the longitudinal slit 14 extends from the beveled tip 13 for 50% of the length of the needle 11.

In the embodiment of Figs. 1A and 1B, the beveled tip 13 may form a predetermined angle with respect to a longitudinal axis x-x of the hollow needle 11. The angle may be for example comprised between from 10° to 45°, depending on the type of tissue. In Fig. 1A, for example, the predetermined angle with respect to the longitudinal axis x-x is of 30°. In Fig. 1B, for example, the predetermined angle with respect to the longitudinal axis x-x is of 30°.

When the needle 11 comprises a longitudinal slit 14 extending from the beveled tip 13 greater than 50% of the length of the needle 11, such as in the embodiment of Fig. 1B, the needle 11 may comprise a mechanical stop 15 located at an intermediate portion of the slit 14, for example at half the length of the slit 14, thus defining two slit portions 14a, 14b. In alternate embodiments, the stop 15 is located at a distance f starting from the beveled tip 13. The stop 15 may be useful to limit the extent to which a slider, which will be described in more details in the following, may slide along the slit 14 of the needle 11.

In the embodiment of Fig. 1C, the needle 11 may further comprise a ring, designated by reference 16, which may be arranged in any region other than the region including the longitudinal slit 14, for example located at half length of the needle. The ring 16 is intended to cooperate with an element of a device for suturing an incision in tissues of a patient, for setting the needle 11 at a fixed tissue penetration depth corresponding to a penetration depth wished by a surgeon. This element will be described herebelow in connection with embodiments of a device for suturing an incision in tissues of a patient.

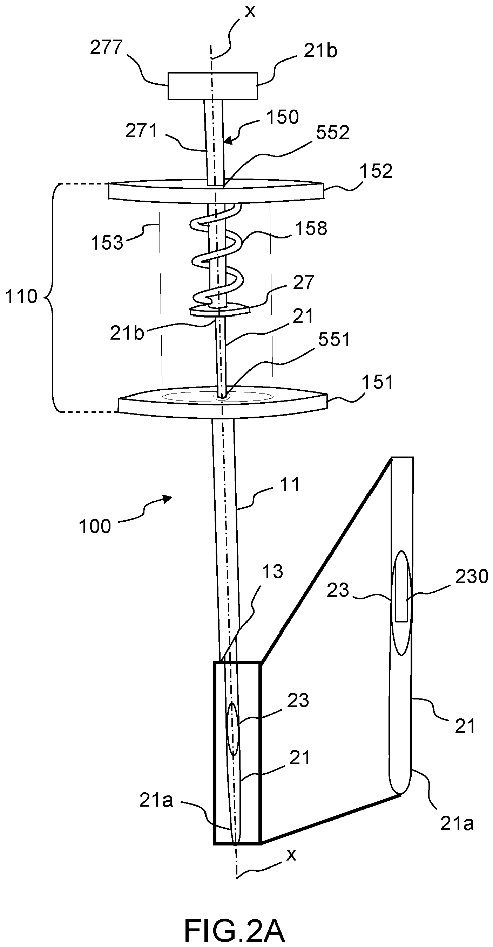

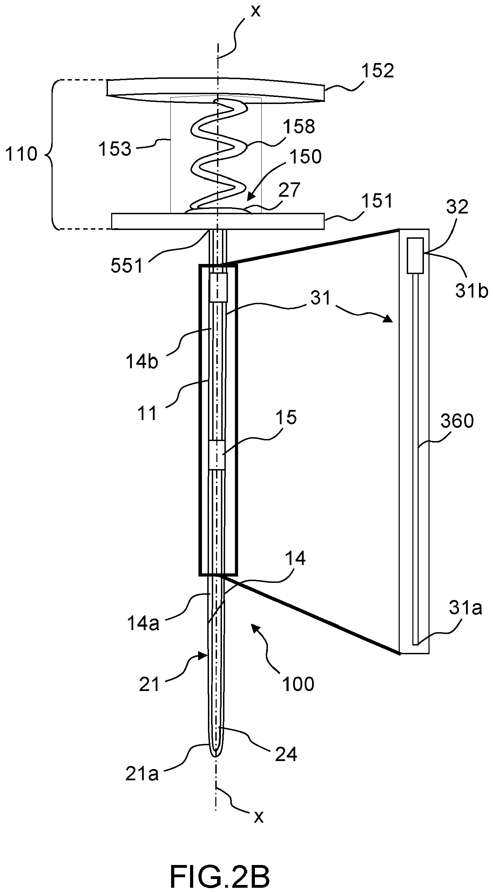

As to a device for suturing an incision in tissues of a patient, a first and a second embodiment thereof, both designated by reference number 100, are shown in Figs. 2A and 2B, respectively.

In the first embodiment of Fig. 2A, which may be suitable for a manual operation, the device 100 comprises the above-mentioned needle 11, a first cylindrical element 21 configured to be received within the hollow needle 11 in a slidable manner, and guiding means 150 for guiding the first cylindrical element 21 within the hollow needle 11 in a slidable manner between two positions. The first cylindrical element 21 may comprise an open stylet, for example including a proximal end 21b provided with a disc 27 and a smooth, for example rounded, closed distal end 21a for protecting the tissues from injury by the beveled tip 13 of the needle 11. The smooth distal end 21a of the first cylindrical element 21 is intended to contact the tissues of the patient in order to avoid any risk that the needle 11 causes any wound when the device 100 is used. According to one or more embodiments, the first cylindrical element 21 comprises a hole 23 of a predetermined length which may be provided with a flexible lamella 230 suitable for expelling a suture thread carried by the first cylindrical element 21 when the device 100 is used.

In the embodiment of Fig. 2A, the hollow needle 11 comprises a proximal portion 110 comprising two trays 151, 152 parallel to one another and to a direction orthogonal to the direction of the longitudinal axis x-x, at a fixed distance, and a hollow cylinder 153 connecting the two trays 151, 152 to each other. The two trays 151, 152 comprise respective through holes 551, 552 configured to receive the first cylindrical element 21. The hollow cylinder 153 may be a transparent cylinder or a non-transparent cylinder. This proximal portion 110 is suitable to be used as a handle for the surgeon and may be used by a single hand.

In the embodiment of Fig. 2A, the guiding means 150 comprises a piston 271, attached to the disc 27 of the first cylindrical element 21 and extending along the axis x-x, and a tray 277 arranged at the end of the piston 150 orthogonally to the axis x-x. The guiding means 150 is connected to the first cylindrical element 21 through the disc 27, thus assuring a full junction between the first cylindrical element 21 and the guiding means 150. The guiding means 150 further comprises a spring 158 or any other suitable elastic means for loading the piston 271 and thus the first cylindrical element 21. A first end of the spring 158 of the guiding means 150 is fixed to the disc 27 of the first cylindrical element 21, and a second end of the spring 158 of the guiding means 158 is fixed to one of the trays 151, 152, for example, as in Fig. 2A, to tray 152. When the second end of the spring 158 is connected to the tray 152, as shown, the spring 158 offers a thrust resistance when the guiding means 150 is pushed.

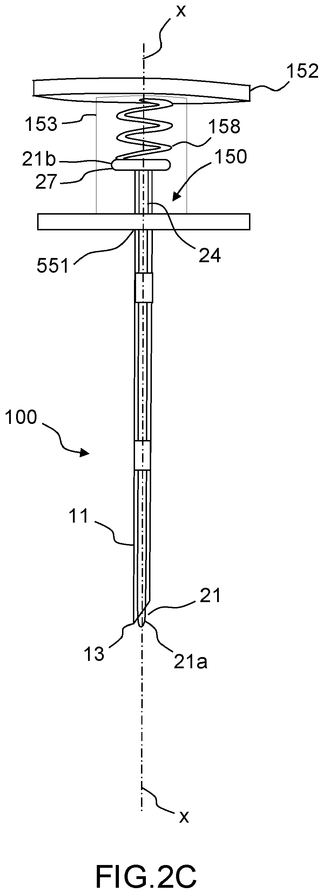

Turning to the second embodiment of Figs. 2B and 2C of a device 100 for suturing an incision in tissues of a patient, which may also be suitable for a manual operation, Fig. 2B shows the device 100 in a resting position, whereas Fig. 2C shows the device 100 in a cutting position, as explained herebelow.

In the embodiment of Fig. 2B, the device 100 comprises a hollow needle 11, a first cylindrical element 21 configured to be received within the hollow needle 11 in a slidable manner, the first cylindrical element 21 comprising a smooth distal end 21a for protecting tissue from injury by the beveled tip 13, guiding means 150 for sliding the first cylindrical element 21 within the hollow needle 11, and a second cylindrical element 31 configured to slide within the first cylindrical element 21, which can optionally be automatically operated. However, the device 100 according to the second embodiment, may be operated in manual manner, as in the case of the first embodiments.

In the embodiment of Fig. 2B, the second cylindrical element 31 comprises an elongated body 360 which comprises a proximal end 31b and a distal end 31a. The proximal end 31b may comprise a slider 32 configured to be blocked by the mechanical stop 15. This second cylindrical element 31 may be manually operated by acting on the slider 32, namely by displacing the slider 32.

Compared with the previous described embodiment shown in Fig. 2A, wherein the device 100 for suturing an incision in tissues of a patient comprises the hollow needle 11 according to the embodiment of Fig. 1A, the device 100 for suturing an incision in tissues of a patient of the embodiment shown in Fig. 2B comprises the hollow needle 11 according to the embodiment of Fig. 1B hereabove described. Moreover, the first cylindrical element 21 comprises a longitudinal slit 24 extending from the smooth distal end 21a for a predetermined length corresponding to 50% or 100% of the length of the first cylindrical element 21. The slit 24 is configured to house the second cylindrical element 31 and to be a guiding track for the second cylindrical element 31. In this embodiment, the proximal portion 110 comprises one through-hole 551 suitable to receive the first cylindrical element 21. In the embodiment of Fig. 2B, the guiding means 150 comprises the disc 27 of the first cylindrical element 21 and a spring 158 or any other suitable elastic means for loading the disc 27. A first end of the spring 158 of the guiding means 150 is fixed to the disc 27 of the first cylindrical element 21, and a second end of the spring 158 of the guiding means 150 is fixed to the tray 152.

A use of the device 100 according to the second embodiment is described in the following. The device 100 according to the second embodiment is shown in Fig. 2B in a resting position, prior to the use of the device, with the first cylindrical element 21 slid outside of hollow needle 11, thus preventing undesired cutting. This resting position is obtained by depressing the spring 158.

Prior to cut tissue layers, the device 100 and in particular the distal end 21a of the first cylindrical element 21 is put into contact with the body (not shown). During use, the surgeon will push the distal end 21a of the first cylindrical element 21 into the skin. Under pressure, the first cylindrical element 21, whose distal end 21a protrudes from the beveled tip 13, retracts and causes compression of the spring 158, allowing the beveled tip 13 to penetrate the tissues of the abdominal wall. This corresponds to the cutting position as shown in Fig. 2C, although the body is not shown in this figure. Once the tissues are cut by the hollow needle 11, no more resistance is offered to the spring 158, which passes to its resting position, thus moving the first cylindrical element 21 outside hollow needle 11, avoiding any damages to organs. The device 100 is thereby in position to anchor a suture thread.

Once the device 100 is in position to anchor a suture thread, the surgeon may perform the following actions, depending on the embodiment of the device.

With reference to the embodiment of the device shown in Fig. 2A, the surgeon, by pushing the tray 277, expels a suture thread previously inserted in the hole 23 of the first cylindrical element 21 for anchoring said suture thread in tissue layers.

With reference to the embodiment of the device shown in Fig. 2B, the surgeon, by moving the slider 32 towards the proximal end 21a of the first cylindrical element 21, expels a suture thread previously inserted in the longitudinal slit 14 of the first cylindrical element 21 for anchoring said suture thread in tissue layers.

In the embodiments in which the needle 11 comprises a ring 16, such as the embodiment of Fig. 1C, the device 100, be it in the form as shown in Fig. 2A or as shown in 2B or in any other embodiment, may further preserve the tissues and organs located below a predetermined tissue penetration depth and improve the safety during use of the needle by a surgeon. One embodiment of the ring 16 is shown in Fig. ID. In this figure, the ring 16 comprises a hollow cylinder 161 having a truncated cone shape. The hollow cylinder 161 is intended to cooperate with a device or a system for suturing an incision in tissues of a patient as described above and further comprising a hollow cylinder 164 having a truncated cone shape complementary to the truncated cone shape of the ring 16. According to one or more embodiments, the ring 16 may comprise a slit (not shown) to facilitate the thread displacement along the longitudinal slit 14.

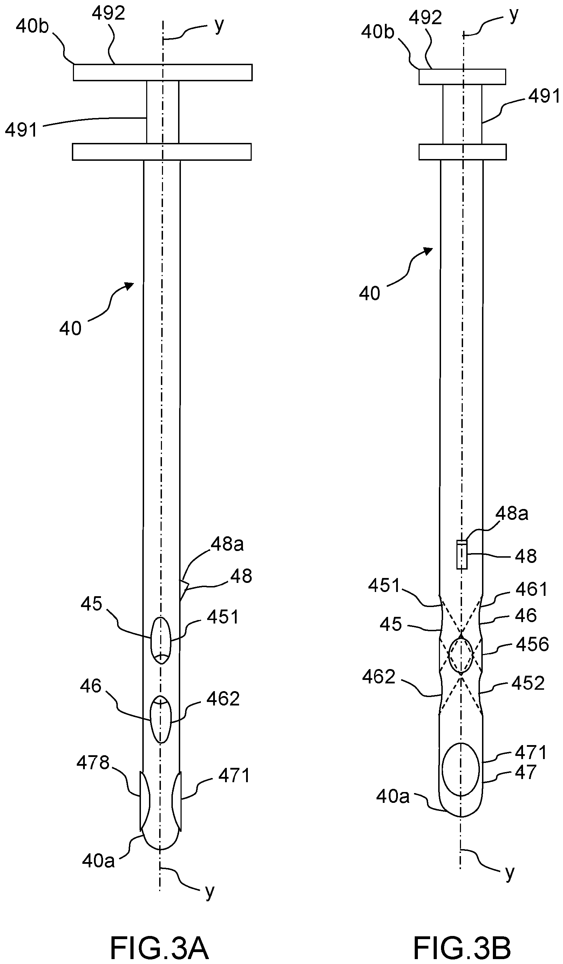

According to one or more embodiments of a device for suturing an incision in tissues of a patient, the device may further comprises, in addition to the exemplary components described above with reference to the embodiments of Figs. 2A and 2B, also a tubular hollow body, which is designated by the reference 40 in Fig. 3A and Fig. 3B.

The tubular hollow body 40 comprises at least one guide channel, for example two guide channels 45, 46 for guiding a hollow needle 11 of the device 100 therethrough at a predetermined angle, for example of 10°, with respect to a longitudinal axis y-y of the tubular hollow body 40. In the embodiment shown in Fig. 3B, the two guide channels 45, 46 cross each other by sharing a common section 456. Each of the two guide channels 45, 46 extends from a first opening 451, 461 to a second opening 452, 462 in a portion proximal to a distal end 40a of the tubular hollow body 40.

The tubular hollow body 40 shown in Fig. 3A and Fig 3B further comprises a means for uplifting one or more tissue layers. The means for uplifting one or more tissue layers comprises inflatable means 47 which are located at the distal end 40a of the tubular hollow body 40. The inflatable means 47 comprises two balloons 471, 472, for example located on opposite sides of the distal end 40a of the tubular hollow body 40. When inflatable means are used, as in this embodiment, the tubular hollow body 40 may further comprise pressurizing means 491, 492, for example located at a proximal end 40b of the tubular hollow body 40 for inflating the inflatable means 47. An example of pressurizing means 491, 492 according to the embodiment of Fig. 3A and Fig. 3B may comprise a piston 492 which may be manually activated and compress air trapped in the volume where the piston 492 moves.

In Fig 3A and Fig 3B, the two balloons 471, 472 are not inflated. To inflate the two balloons 471, 472, the piston 492 is activated, thereby injecting the volume of air trapped under the piston 492 into the two balloons 471, 472 via the tubular hollow body 40.

Further, as shown in Figs 3A and 3B, the tubular hollow body 40 may further comprise a latch 48 arranged on the external wall of the tubular hollow body 40, for example in a retractable manner. During use, the latch 48 may be deployed by passing the tubular hollow body 40 through a trocar. For example, the trocar may lay on a shoulder 48a of the latch 48, which permits to uplift the trocar 80 when uplifting the tubular hollow body 40. To equilibrate forces of the trocar 80 laying on the shoulder 48a, two or four latches 48 may be used.

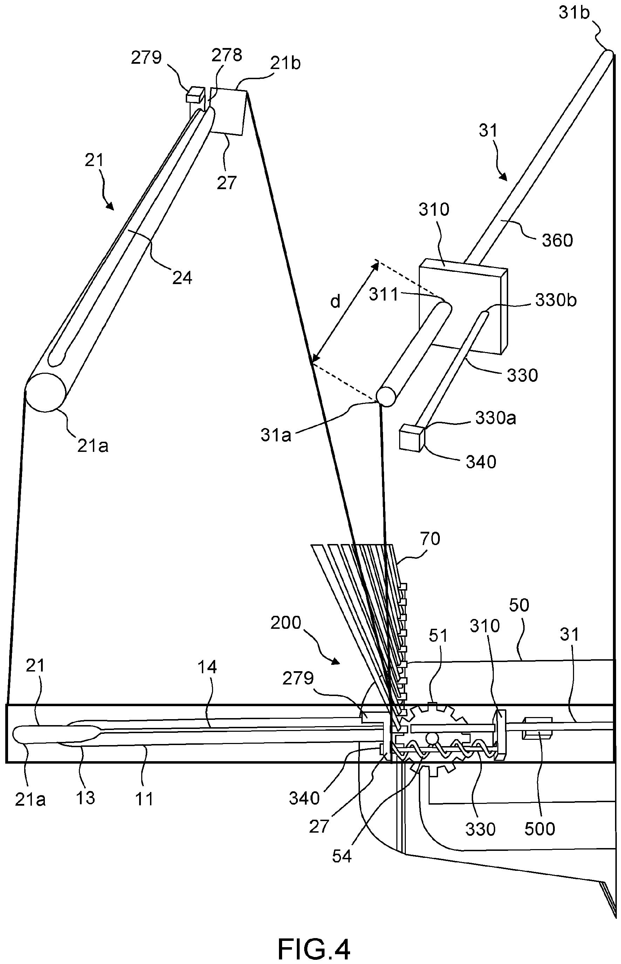

An embodiment of a system for suturing an incision in tissues of a patient, designated by reference 200, is shown in Fig. 4. The system 200 described hereafter comprises the embodiment of the device 100 comprising the second cylindrical element 31, and a means 50 for automatically sliding the second cylindrical element 31 within the first cylindrical element 21. In the embodiment shown in Fig. 4, the means 50 for automatically sliding the second cylindrical element 31 within the first cylindrical element 21 comprises a projection mechanism for projecting the second cylindrical element 31 housed in a slidable manner within the first cylindrical element 21 for surgical suture gripping. In the figure, the projection mechanism comprises a tag gun similar to a tag gun for using and reloading with tags in the clothes industry. The tag gun comprises a gear 51 for converting an initial movement of a gun trigger (not shown) into a sliding movement of the second cylindrical element 31 within the first cylindrical element 21, as well as a spring 54 for returning the second cylindrical element to a resting position. A first end of the spring 54 is fixed to the first cylindrical element 21, and a second end of the spring 54 is fixed to the second cylindrical element 31. Further, the means 50 for automatically sliding the second cylindrical element 31 within the first cylindrical element 21 comprises at least one guiding element 500 for guiding the second cylindrical element 31. From a cinematic point of view, the second cylindrical element 31 and the means 50 form a slide connection through the at least one guiding element 500.

Compared with the first cylindrical element 21 described in connection with the embodiment shown in Fig. 2B, the first cylindrical element 21 of the embodiment of the system 200 further comprises a groove 278 and a lug 279 provided in the disc 27 of the first cylindrical element 21. According to other embodiments, the disc 27 may comprise a hole instead of the groove 278 provided in the embodiment of Fig. 4. In both cases, the hole and the groove 278 of the disc 27 are configured to let an element of the second cylindrical element 31 to move therethrough, namely a stem described in more detail in the following. Further, the disc 27, in the embodiment shown, has a square shape. However, other shapes are possible.

Compared with the second cylindrical element 31 described in connection with the embodiment shown in Fig 2B, the second cylindrical element 31 of the embodiment of the system 200 may include an elongated body 360 solidarized with an intermediate part 310 located at a length d of the elongated body 360. The intermediate part 310 may be in the form of a first tray, for example of parallelepipedic structure, in which a hole 311 is defined to receive the second cylindrical element 31. The intermediate part 310 may further comprise a stem 330 comprising a proximal end 330a and a distal end 330b and configured to slide in the hole or the groove 278 of the disc 27 as well as in the slit 24. The distal end 330b of the stem 330 may be attached to the tray and the proximal end 330a may comprise a second tray 340. In the example shown in Fig. 4, the second tray 340 also has a parallelepipedic structure. In the embodiment shown in Fig. 4, the intermediate part 310 is fixed to the system 200, thus serving as a guiding element for the second cylindrical element 31 longitudinally sliding through the hole 311.

In the example shown in Fig. 4, the means 50 further comprises a plurality of suture threads 70.

During use, the surgeon will push the distal end 21a of the first cylindrical element 21 of the device 200 into the skin. Under pressure, the first cylindrical element 21, whose distal end 21a protrudes from the beveled tip 13, retracts and causes compression of the spring 54. The first end of the spring 54 is fixed to the first cylindrical element 21, and in particular to the disc 27 of the first cylindrical element 21, while the second end of the spring 54 is fixed to the second cylindrical element 31, in particular to the intermediate part 310. Therefore, the spring 54 moves back, allowing the beveled tip 13 to penetrate the tissues of the abdominal wall.

Once the tissues are cut by the hollow needle 11, no resistance is offered any longer to the spring 54, which passes to its resting position, thus moving the first cylindrical element 21 outside the hollow needle 11, and avoiding any damages to the organs. The device 200 is thereby in position to anchor a suture thread.

Once the device 200 is in position to anchor a suture thread, the gear 51 brings down a suture thread 70 of the plurality of suture threads between the entrance of the slit 24 and the distal end 31a of the second cylindrical element 31. By sliding through the hole 311 of the intermediate part 310, the second cylindrical element 31 hits the suture thread 70 localized in front of the slit 24. As a consequence, the suture thread 70 is inserted in the slit 24 of the first cylindrical element 21, thus moving the suture thread 70 at the distal end 11a of the hollow needle 11 prior to be anchored in a tissue layers.

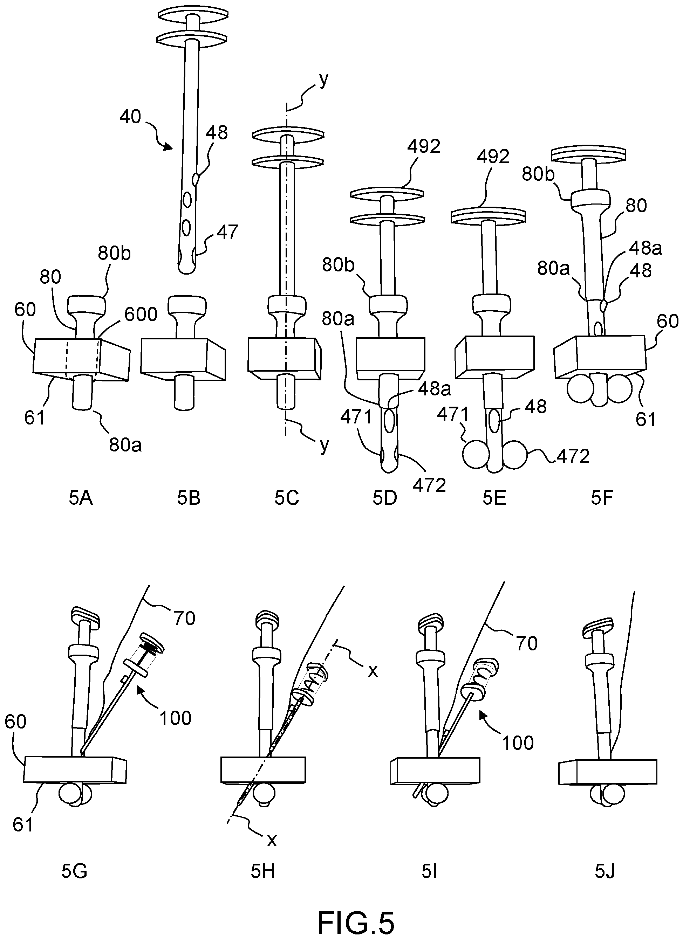

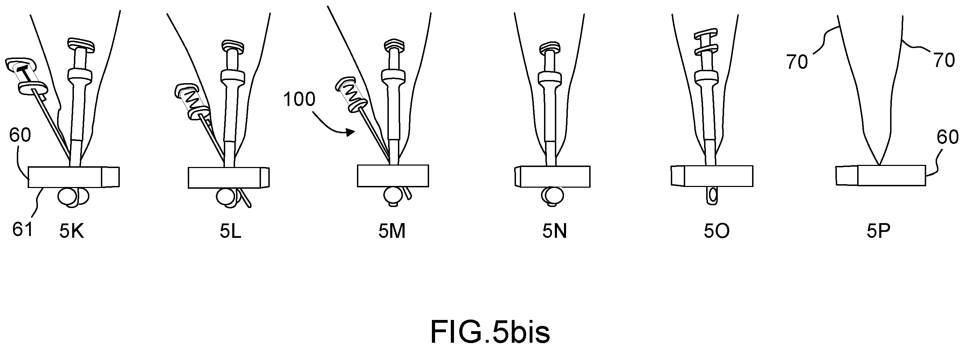

Figs. 5 and 5bis illustrate a method for suturing an incision 600 in a tissues of a patient. In the exemplary method schematically shown in Figs. 5 and 5bis, a trocar 80 is initially inserted in the incision 600 in a tissue 60 (scheme 5A). The tissue 60 delimits a patient or an animal body. A tubular hollow body 40 of the device 100 according to embodiments previously described is introduced inside the trocar 80, which also serves as a guide for the tubular hollow body 40, and penetrates beyond the tissue 60 inside the patient body, as shown in schemes 5B-5D.

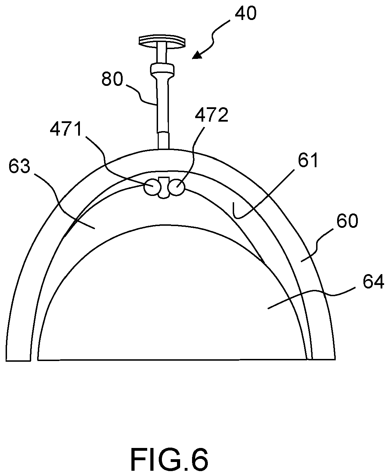

After the tubular hollow body 40 reaches an operational position set by the deployment of the latch 48 by contacting the shoulder 48a of the latch 48 with a distal end 80a of the trocar 80, the tubular hollow body 40 is then uplifted until the balloons 471, 472, which have been inflated by pushing the piston 492, contact an innermost layer 61 of said tissue 60 in a proximity of the incision 600 to suture (scheme 5F). Subsequently, the tissue 60 can be uplifted by pulling the tubular hollow body 40 so as to create a safety space 63 between the tissue 60 and one or more internal organs 64, as shown in Fig. 6. Once the tissue 60 is uplifted, the tissue 60 is in position to allow a suturing of the incision 600.

The device 100 is then inserted within a first guide channel of the two guide channels 45, 46 of the tubular hollow body 40 (schemes 5G-5I). At least one suture thread 70 is carried by the device 100 or the system 200. Once the device 100 reaches an anchoring position, the guiding means 150 of the device 100 is used in order to anchor the suture thread 70 in the tissue 60 (scheme 5J). This operation is repeated after the device 100 is inserted within a second guide channel of the two guide channels 45, 46 of the tubular hollow body 40 (schemes 5K-5M). A second suture thread 70 is then anchored in the tissue layers 60 (scheme 5N).

After anchoring suture thread 70, the balloons 471, 472 are deflated by pulling out the piston 492, and the tubular hollow body 40 and the trocar 80 may be pulled out from the incision (scheme 5O-5P), thereby allowing to suture the incision 600.

Although this is not shown in the figures, in an analogous manner, a tubular hollow body 40 of the system 200 according to embodiments previously described may be introduced inside the trocar 80 with reference to the scheme 5A. In this case, the guiding means 50 of the system 200 is used in order to anchor the suture thread 70 in the tissue 60.

Modifications and variations as would be apparent to a skilled addressee are deemed to be within the scope of the present invention. Further, it should be appreciated that the scope of the invention is not limited to the scope of the embodiments disclosed. By way of example, the needle, device, system and method according to the present disclosure may be suitable to suture any type of incision in human or animal bodies.

## Claims

1. A hollow needle (11) comprising a proximal end portion (11b) and a distal end portion (11a), wherein the distal end portion (11a) comprises a beveled tip (13) for cutting through tissues (60) of a patient and a longitudinal slit (14) extending from the beveled tip (13) for a predetermined length (L).

2. A device (100) for suturing an incision in tissues (60) of a patient, the device (100) comprising:
the hollow needle (11) according to claim 1,
a first cylindrical element (21) configured to be received within the hollow needle (11) in a slidable manner, and
a guiding means (150) for sliding the first cylindrical element (21) within the hollow needle (11),
wherein the first cylindrical element (21) comprises a smooth distal end (21a) for protecting tissue from injury by the beveled tip (13).

3. The device (100) according to claim 2, wherein the first cylindrical element (21) is hollow and the device (100) further comprises a second cylindrical element (31) configured to slide within the first cylindrical element (21) and optionally to be automatically operated.

4. The device (100) according to claim 2 or claim 3, further comprising a tubular hollow body (40) comprising at least one guide channel (45, 46) for guiding the hollow needle (11) therethrough at a predetermined angle with respect to a longitudinal axis (y-y) of the tubular hollow body (40), the at least one guide channel (45, 46) extending from a first opening (451, 461) to a second opening (452, 462) of the tubular hollow body (40) in a portion proximal to a distal end (40a) of the tubular hollow body (40).

5. The device (100) according to claim 4, wherein the device (100) comprises two guide channels (45, 46).

6. The device (100) according to claim 4 or claim 5, wherein the tubular hollow body (40) is configured to receive a surgical instrument.

7. The device (100) according to any one of claims 2-6, further comprising a suture thread (70) comprising one end having a T-shape.

8. The device (100) according to any one of claims 4-7, further comprising a means for uplifting one or more tissue layers (60).

9. The device (100) according to claim 8, wherein the means for uplifting one or more tissue layers (60) comprises an inflatable means (47) located at the distal end (40a) of the tubular hollow body (40).

10. The device (100) according to claim 9, wherein the inflatable means (47) comprises two balloons (471, 472) located on opposite sides of the distal end (40a) of the tubular hollow body (40).

11. The device (100) according to claim 9 or claim 10, further comprising pressurizing means (491, 492) located at a proximal end (40b) of the tubular hollow body (40) for inflating the inflatable means (47).

12. The device (100) according to any one of claims 2 to 11, further comprising a trocar (80) receiving the device (100).

13. The device (100) according to claim 12, further comprising a latch (48) arranged on the tubular hollow body (40) in a retractable manner.

14. System (200) for suturing an incision in tissues of a patient, comprising:
the device (100) of any one of claim 3 or claims 4-13 when dependent from claim 3, and
a means (50) for automatically sliding the second cylindrical element (31) within the first cylindrical element (21).
